# EUROPEAN PATENT APPLICATION

(11) **EP 4 830 656 A1**
(43) Date of publication of application: **28.10.2026**
(21) Application number: 24909998.7
(22) Date of filing: 10.09.2024
(51) Int. Cl.: A61B 34/10, A61B 17/34, A61B 34/20

(54) **INTELLIGENT PUNCTURE NAVIGATION SYSTEM AND METHOD**

(30) Priority: 28.12.2023 CN 202311824637
(71) Applicant: Jiangsu Chunci Medical Treatment Technology Co., Ltd., Nanjing, Jiangsu 210001 (CN)
(72) Inventor: YU, Jiangping, Nanjing, Jiangsu 210001 (CN); ZHENG, Suyang, Nanjing, Jiangsu 210001 (CN); LIU, Chen, Nanjing, Jiangsu 210001 (CN); LI, Guodong, Nanjing, Jiangsu 210001 (CN); ZHANG, Lele, Nanjing, Jiangsu 210001 (CN)
(74) Representative: Seemann & Partner Patentanwälte mbB
(86) International application number: PCT/CN2024/117954
(87) International publication number: WO 2025/139052

(57) **Abstract**

Provided is an intelligent puncture navigation system and method. The system comprises a medical imaging device (1), an upper computer (2), a positioning device (3), and an intelligent puncture needle (4). The medical imaging device (1) is configured to acquire a three-dimensional scanning image of tissues to be punctured; the upper computer (2) is configured to determine puncture information according to the three-dimensional scanning image; the positioning device (3) is configured to form a mark on the skin surface of the area to be punctured according to the puncture information; the intelligent puncture needle (4) is configured to perform puncturing according to the mark and the puncture information and acquire real-time angle information of the puncture needle; the upper computer (2) is further configured to monitor the intelligent puncture needle (4) according to the real-time angle information. The intelligent puncture navigation system and method can achieve the navigation function without image registration, thereby avoiding puncture deviation or failure caused by image registration errors. The system and method offer flexible use and improve the puncture efficiency and accuracy.

## Description

This application claims priority to the Chinese Patent Application No. 202311824637.8, filed with the China National Intellectual Property Administration on December 28, 2023 and entitled "INTELLIGENT PUNCTURE NAVIGATION SYSTEM AND METHOD", which is incorporated herein by reference in its entirety.

### TECHNICAL FIELD

The present disclosure relates to the field of medical surgical navigation, and in particular, to an intelligent puncture navigation system and method.

### BACKGROUND

When a lesion inside the human body requires a biopsy or treatment via puncture, a computed tomography (CT) scanner is often used to determine the orientation between the lesion and the skin marker point, as well as the depth of the puncture needle. Specifically, the CT scanner first measures the position of the lesion in a transverse section of the human body, selects an optimal needle insertion position and angle in the transverse section, and determines the precise positions of the lesion and the puncture needle by using the three-dimensional reconstruction of the insertion plane, insertion angle, and insertion depth. Although the CT scanner can accurately determine the three-dimensional insertion angle and depth, the puncture procedure is typically performed after the patient is moved out of the CT scanning plane. When the patient is away from the CT scanner, the physician can only determine a general insertion direction based on personal judgment to perform puncture, and then use CT scanning for confirmation. Due to numerous human factors, inaccurate needle insertion often occurs, affecting the precision of the treatment. Sometimes repeated needle insertions are required, and in severe cases, it can lead to mispuncture, causing great pain and risk to the patient.

To address the above issues, puncture surgical navigation systems have emerged. A puncture surgical navigation system consists of positioning, three-dimensional image reconstruction for stereotaxis, and a robotic arm. The operator manipulates the robotic arm of the system based on pre-stored image data on a monitor. The monitor can display the insertion path of the puncture needle, improving puncture accuracy and safety. According to different positioning methods, puncture surgical navigation systems in some scenarios are divided into two types: optical navigation systems and magnetic navigation systems. These two types of puncture surgical navigation systems cannot display the angle information of the puncture needle, but only determine the angle information of the robotic arm to estimate the angle of the puncture needle, thus failing to display the angle of the puncture needle in real time. During the puncture procedure, changes in the posture of the patient can also alter the puncture accuracy. Although a robotic arm can guide the puncture direction and ensure no deviation of the puncture angle, it is extremely inflexible in adjustment. Puncture generally requires the patient to remain still, but in reality, the human body cannot be completely stationary. Internal organs such as the intestines, lungs, and trachea are constantly moving. Therefore, adjustments to the puncture angle are often required during the puncture procedure. When part of the puncture needle has entered the body, adjusting the puncture angle is not simply changing from one angle to another. Due to factors such as muscle clamping, needle deformation, and needle rigidity, if an adjustment of, for example, 15 degrees is needed, the needle may need to be pressed down or pushed up by 30 or even 50 degrees to achieve the intended 15-degree adjustment upon natural rebound of the puncture needle. Therefore, a robotic arm that only guides a fixed puncture direction is inconvenient in use and cannot be adjusted in real time as needed. Moreover, current puncture surgical navigation systems are expensive and difficult to popularize.

### SUMMARY

An objective of the present disclosure is to provide an intelligent puncture navigation system and method that realize functions of intelligent navigation and active puncture guidance, avoid puncture deviation or failure caused by image registration errors, are flexible in use, and improve puncture efficiency and accuracy.

To achieve the foregoing objective, embodiments of the present disclosure provide the following technical solutions:
An intelligent puncture navigation system is provided, including a medical imaging device (1), a host computer (2), a positioning device (3), and an intelligent puncture needle (4). The medical imaging device (1) is configured to acquire a three-dimensional scanned image of a target puncture tissue; the medical imaging device (1) may be a CT or magnetic resonance imaging (MRI) device. The host computer (2) is configured to determine puncture information according to the three-dimensional scanned image, where the puncture information may include a puncture point position, a puncture angle, and a needle insertion depth. The positioning device (3) is configured to form a mark on a skin surface of the target puncture tissue according to the puncture information, where the mark may be a needle insertion point position. The intelligent puncture needle (4) is configured to perform puncture according to the mark and the puncture information, and acquire real-time angle information of the intelligent puncture needle (4). The host computer (2) is further configured to monitor the intelligent puncture needle (4) according to the real-time angle information. The positioning device (3), the intelligent puncture needle (4), and the medical imaging device (1) share a coordinate system. The positioning device (3) may include a first rail (31) and a second rail (32). The first rail (31) and the second rail (32) are arranged directly above a scanning table (11) of the medical imaging device (1), where the first rail (31) is arranged parallel to a length direction of the scanning table (11); the second rail (32) is perpendicular to the first rail (31) and disposed on the first rail (31), capable of sliding along the first rail (31) when driven by the first rail (31). A laser emitter (33) facing the scanning table (11) is arranged at a lower side of the second rail (32), and the laser emitter (33) is capable of moving along the second rail (32) when driven by the second rail (32). The positioning device (3) may further include a microcontroller and a wireless communication module. The wireless communication module and the laser emitter (33) are electrically connected to the microcontroller, and the microcontroller is configured to control movement of the first rail (31) and the second rail (32). The host computer (2) communicates wirelessly with the microcontroller and transmits coordinate information of a puncture point to the microcontroller. The microcontroller then controls the movement of the first rail (31) and the second rail (32) according to the coordinate information of the puncture point, adjusting the laser emitter (33) to a position corresponding to the coordinate information. At this time, the microcontroller controls the laser emitter (33) to emit laser light, and the laser light hits a human body, marking the puncture point position. The intelligent puncture needle (4) may include a sleeve (43) detachably sleeved outside a needle hub (412) of a puncture needle body (41). A flexible printed circuit board (FPCB) is arranged inside a side wall of the sleeve (43) along a length direction of the sleeve (43). At least two laser receivers (431) are disposed on the FPCB. Corresponding to the laser receivers (431), the sleeve (43) is provided with holes. A second pogo pin plug (432) is arranged at an end of the sleeve (43). The second pogo pin plug (432) is electrically connected to the FPCB. Correspondingly, a second pogo pin receptacle (427) is arranged at a lower side of a housing (42), and is electrically connected to a circuit board. The medical imaging device (1) emits a laser beam from a set zero position. The laser receivers (431) on the intelligent puncture needle (4) detect the laser beam. When two laser receivers (431) located on a same straight line both detect the laser beam, a current position of the puncture needle body is set as a zero point of the puncture needle body. When the intelligent puncture needle (4) is powered on, the second pogo pin plug (432) and the second pogo pin receptacle (427) are electrically connected and communicate with each other. When receiving laser signals, the laser receivers (431) transmit the laser signals to the host computer (2) via a Bluetooth module. When simultaneously receiving the laser signals from the two laser receivers (431) located on the same straight line, the host computer (2) sets a current position of the puncture needle body (41) as a zero point position, achieving automatic registration with the medical imaging device (1). The intelligent puncture navigation system may further include a puncture needle fixation device (5). The puncture needle fixation device (5) may include a silicone fixation plate (51), an adjustment device (52), and a hook (53). The adjustment device (52) may include an adjustment cord (521) and an adjustment buckle. The adjustment buckle may include a lock body (522). One end of the adjustment cord (521) is fixedly connected to the lock body (522) and passes through the lock body (522) to connect to the silicone fixation plate (51). Another end of the adjustment cord (521) passes through the lock body (522) and is adjustably connected to the lock body (522). One end of the hook (53) is slidably connected to a bending portion of the adjustment cord (521). The silicone fixation plate (51) supports the puncture needle body (41) through gravity and friction with skin. The hook (53) is configured to hook the puncture needle body (41). The hook (53) and the silicone fixation plate (51) are connected via the adjustment device (52), where the adjustment device (52) is configured to adjust a distance between the hook (53) and the silicone fixation plate (51) and support the hook (53), thereby adjusting a fixation angle of the puncture needle body (41).

An intelligent puncture navigation method is provided, applied to the intelligent puncture navigation system. The intelligent puncture navigation method includes: establishing an OC coordinate system based on a medical imaging device, and establishing an OL coordinate system based on a positioning device, where an x-axis direction and a z-axis direction of the OC coordinate system are the same as an x-axis direction and a z-axis direction of the OL coordinate system; determining a mapping relationship from the OC coordinate system to the OL coordinate system; acquiring a three-dimensional scanned image of a target puncture tissue using the medical imaging device; determining puncture information by using a host computer based on the three-dimensional scanned image, where the puncture information includes a puncture point position, a puncture angle, and a needle insertion depth; establishing an OP coordinate system based on a position of the target puncture tissue, and determining a mapping relationship from the OP coordinate system to the OC coordinate system, where an origin of the OP coordinate system is located at a needle insertion point position, and the needle insertion point position is a position where the positioning device forms a mark on a skin surface of the target puncture tissue according to the puncture information; determining a mapping relationship from the OP coordinate system to the OL coordinate system based on a mapping relationship from the OL coordinate system to the OC coordinate system and the mapping relationship from the OP coordinate system to the OC coordinate system; establishing an OU coordinate system based on a position of the target puncture tissue in a virtual space, and determining a mapping relationship from the OC coordinate system to the OU coordinate system, where an origin of the OU coordinate system is a needle insertion point position on the target puncture tissue in the virtual space; establishing a one-dimensional ON coordinate system based on an intelligent puncture needle, wherein an origin of the one-dimensional ON coordinate system is a needle tip position of the puncture needle; performing a puncture process simulation in the virtual space based on the puncture information to determine a virtual puncture needle tip position of the puncture needle; determining real-time angle information based on the virtual puncture needle tip position of the puncture needle; and guiding puncture of the intelligent puncture needle based on the real-time angle information.

According to the specific embodiments provided in the present disclosure, the following technical effects are disclosed: After obtaining puncture information via a medical imaging device, the intelligent puncture navigation system of the present disclosure can automatically mark a puncture point position on the human body through a positioning device, obtain real-time angle information of the puncture through an intelligent puncture needle, and monitor the puncture angle in real time, thereby guiding key information during the puncture, namely, the puncture point position and the puncture angle, to accurately guide the puncture. The system has a simple composition. The medical imaging device is standard equipment in current hospitals. By adding a positioning device, an intelligent puncture needle, and a host computer, the puncture navigation function of the present system can be achieved, greatly reducing the diagnosis and treatment costs for hospitals and patients. The system can achieve navigation functionality without undergoing an image registration process, avoiding puncture deviation or failure caused by image registration errors. Furthermore, the use of the intelligent puncture needle is not limited by the constraints of a robotic arm, allowing flexible use and adjustment according to actual conditions during puncture. Moreover, the puncture angle information can be measured in real time according to human body changes during an operation and displayed on the host computer, thereby greatly improving puncture efficiency and accuracy.

### BRIEF DESCRIPTION OF THE DRAWINGS

FIG. 1 is a schematic block diagram of an intelligent puncture navigation system according to one or more embodiments;
FIG. 2 is a schematic diagram of an installation structure of a positioning device in an intelligent puncture navigation system according to one or more embodiments;
FIG. 3 is a schematic structural diagram of a positioning device in an intelligent puncture navigation system according to one or more embodiments;
FIG. 4 is a schematic structural diagram of a positioning device with a frame removed in an intelligent puncture navigation system according to one or more embodiments;
FIG. 5 is a schematic structural diagram of a puncture needle in an intelligent puncture navigation system according to one or more embodiments;
FIG. 6 is a partial structural diagram of FIG. 5 with a housing hidden;
FIG. 7 is a cross-sectional view of FIG. 5;
FIG. 8 is another schematic structural diagram of a puncture needle in an intelligent puncture navigation system according to one or more embodiments;
FIG. 9 is a schematic structural diagram of a housing in FIG. 8;
FIG. 10 is a schematic cross-sectional view of FIG. 9;
FIG. 11 is a schematic structural diagram of an elastic clip according to one or more embodiments;
FIG. 12 is a schematic structural diagram showing the arrangement of a first pogo pin plug and a first pogo pin receptacle according to one or more embodiments;
FIG. 13 is a schematic diagram showing the arrangement of holes on the housing according to one or more embodiments;
FIG. 14 is a schematic structural diagram showing a sleeve disposed on a puncture needle according to one or more embodiments;
FIG. 15 is a schematic structural diagram of a sleeve according to one or more embodiments;
FIG. 16 is a schematic structural diagram showing the arrangement of a second pogo pin receptacle according to one or more embodiments;
FIG. 17 is a schematic structural diagram of a puncture needle fixation device according to one or more embodiments;
FIG. 18 is a cross-sectional view of a lock body according to one or more embodiments;
FIG. 19 is a schematic diagram of an OC coordinate system according to one or more embodiments; and
FIG. 20 is a schematic diagram of an OU coordinate system according to one or more embodiments.

### Reference Numerals:

1-medical imaging device; 11-scanning table; 2-host computer; 3-positioning device; 31-first rail; 311-first frame; 312-first conveyor belt; 313-first motor; 32-second rail; 321-second frame; 322-second conveyor belt; 323-second motor; 33-laser emitter; 34-connecting plate; 4-intelligent puncture needle; 41-puncture needle body; 411-needle core; 412-needle hub; 4121-needle hub rod; 4122-needle hub handle; 41221-first mounting plane; 41222-clamping groove; 42-housing; 421-second mounting plane; 422-clamping block; 423-elastic clip; 424-anti-slip protrusion; 425-first pogo pin plug; 426-first pogo pin receptacle; 427-second pogo pin receptacle; 43-sleeve; 431-laser receiver; 432-second pogo pin plug; 5-puncture needle fixation device; 51-silicone fixation plate; 511-hanging ring; 52-adjustment device; 521-adjustment cord; 522-lock body; 523-adjustment button; 524-fixing hook; 53-hook.

### DETAILED DESCRIPTION OF THE EMBODIMENTS

The technical solutions in the embodiments of the present disclosure are clearly and completely described below with reference to the drawings in the embodiments of the present disclosure. Apparently, the described embodiments are only some rather than all of the embodiments of the present disclosure. All other embodiments obtained by a person of ordinary skill in the art based on the embodiments of the present disclosure without creative efforts shall fall within the protection scope of the present disclosure.

There are two commonly used puncture surgery navigation systems in the prior art, namely, an optical navigation system and a magnetic navigation system. Both navigation systems can match the human body with virtual images and guide puncture, making puncture surgery intuitive and visualized. However, both the optical navigation system and the magnetic navigation system involve a process of registration with virtual images, which is prone to registration errors, thereby affecting the subsequent puncture guidance process and even leading to puncture failure. Moreover, although the robotic arm that guides the puncture can theoretically be adjusted at multiple angles, it is often necessary to adjust the puncture angle during puncture. Since the puncture needle has already partially entered the human body at this time, due to muscle clamping as well as the rigidity and flexibility of the puncture needle, the adjustment process is not simply changing from one angle to another. For example, if an adjustment of 15 degrees is needed, the needle may need to be pressed down or pushed up by 30 or even 50 degrees to achieve the intended 15-degree adjustment upon natural rebound of the puncture needle. Under such circumstances, the robotic arm is inconvenient and inflexible to use. Furthermore, current puncture surgical navigation systems are expensive and costly, but their actual effectiveness is unsatisfactory. Moreover, the registration process between the human body and virtual images generally takes 30 to 40 minutes, which also increases surgery time and risks.

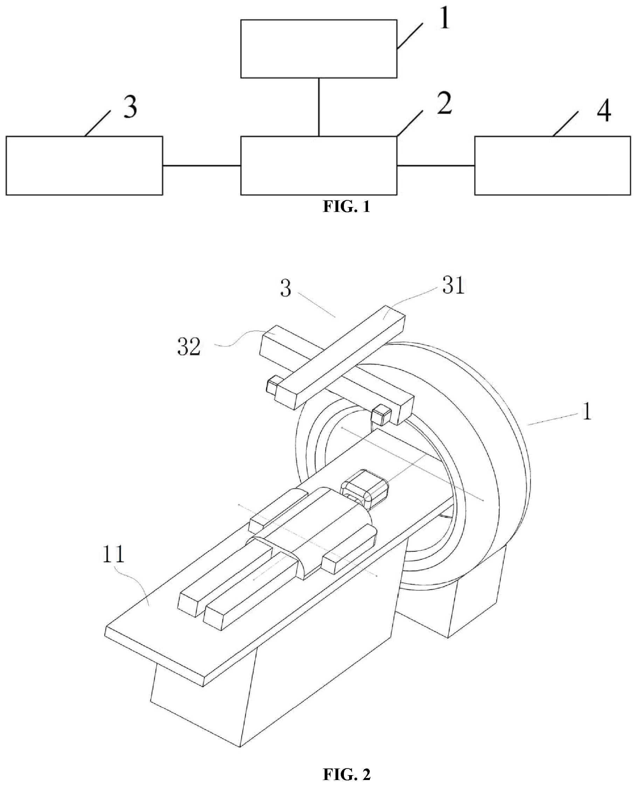

To solve the above problems, in one application example, the present application provides an intelligent puncture navigation system, as shown in FIG. 1, comprising a medical imaging device 1, a host computer 2, a positioning device 3, and an intelligent puncture needle 4.

The medical imaging device 1 is configured to acquire a three-dimensional scanned image of a target puncture tissue. The medical imaging device is mainly a CT or MRI device commonly used in hospitals currently. CT includes conventional CT devices, cone beam CT (CBCT), and the like. Taking CT as an example, scanning is generally performed by a CT device to obtain transverse sectional scanned images of the human body, and three-dimensional scanned images can be reconstructed from the transverse sectional images.

The host computer 2 is configured to determine puncture information according to the three-dimensional scanned image. After the three-dimensional scanned image of the target puncture tissue is obtained by the medical imaging device 1, it is transmitted to the host computer 2, and a doctor, at the host computer side, determines puncture information based on the three-dimensional scanned image. First, a needle insertion plane is determined. A puncture path is determined based on an anatomical structure and a tumor position, avoiding vital organs such as nerves, blood vessels, and bones. Then the puncture information is further determined, mainly including a puncture point position, a puncture angle, and a needle insertion depth.

The positioning device 3 is configured to form a mark on a skin surface of the target puncture tissue according to the puncture information. The host computer 2 communicates with the positioning device 3, and transmits coordinate information of the puncture point to the positioning device 3. The positioning device 3 operates in coordination with the host computer 2, and is primarily configured to form a mark on the skin surface of the target puncture tissue.

The intelligent puncture needle 4 is configured to perform puncture according to the mark and the puncture information, and acquire real-time angle information of the intelligent puncture needle 4. Common puncture needles are provided with scale marks. Therefore, a doctor can observe the needle insertion depth at any time during puncture, but the puncture angle cannot be observed intuitively. By contrast, using the intelligent puncture needle 4 of the present disclosure for puncture, a doctor can obtain the real-time puncture angle. The intelligent puncture needle 4 is formed by integrating smart sensors, such as an angle sensor and a gyroscope, onto an ordinary puncture needle, enabling the acquisition of real-time angle information of the puncture needle during puncture.

The host computer 2 is further configured to monitor the intelligent puncture needle 4 according to the real-time angle information. After the real-time angle information of the intelligent puncture needle 4 is obtained, the information is transmitted to the host computer 2, and the host computer 2 monitors the puncture angle of the puncture needle body 41 according to the real-time angle information.

In use of the intelligent puncture navigation system in the above embodiment, first, a three-dimensional scanned image of the target puncture tissue is obtained by the medical imaging device 1, and then the host computer 2 determines the puncture information based on the three-dimensional scanned image. The puncture information generally includes a puncture point position, a puncture angle, and a needle insertion depth. The system further includes the positioning device 3 that can form a mark on the skin surface of the target puncture tissue according to the puncture information, i.e., mark the position on the human skin based on the information of the puncture point position. Meanwhile, during puncture with the intelligent puncture needle 4, the needle insertion depth can be determined by observing the scale marks on the puncture needle, and the real-time angle information of the puncture needle can also be obtained through the intelligent puncture needle 4 for comparison with the set puncture angle information during puncture, to determine whether the puncture angle deviates from the setting. In addition, the host computer 2 can also monitor the intelligent puncture needle 4 based on the real-time angle information, achieving automatic monitoring of the puncture needle body 41.

After obtaining the puncture information, the intelligent puncture navigation system of the present disclosure can automatically mark a puncture point position on the human body through the positioning device 3, obtain real-time angle information of the puncture through the intelligent puncture needle 4, and monitor the puncture angle in real time, thereby guiding key information during the puncture, namely, the puncture point position and the puncture angle, to accurately guide the puncture. The system has a simple composition. The medical imaging device and the host computer are both standard equipment in current hospitals. By adding the positioning device 3 and the intelligent puncture needle 4, the puncture navigation function of the present system can be achieved, greatly reducing the diagnosis and treatment costs for hospitals and patients. The system can achieve navigation functionality without undergoing an image registration process, avoiding puncture deviation or failure caused by image registration errors. Furthermore, the use of the intelligent puncture needle 4 is not limited by the constraints of a robotic arm, allowing flexible use and adjustment according to actual conditions during puncture. Moreover, the next operation can be determined according to the real-time angle information, greatly improving puncture efficiency and accuracy.

In an embodiment, the host computer 2 is further configured to form a simulation animation on the three-dimensional scanned image based on the real-time angle information. The simulation animation is mainly used to simulate a puncture process of the puncture needle, particularly an angle change process of the puncture needle. The difference between such a simulation animation and simulation animations in puncture surgical navigation systems in the prior art is that the simulation animation of the present disclosure simulates the angle change of the puncture needle based on collected real-time angle information of the puncture needle, and such simulation belongs to real-time monitoring. In contrast, the puncture surgical navigation systems in the prior art realize precise registration between a virtual human body in a simulated image and a real human body, and guide the puncture direction using virtual image data. This is a pre-operative procedure requiring high precision, and is therefore susceptible to slight errors causing severe deviations. In comparison, the real-time simulation adopted in the present disclosure provides far superior guidance.

In an embodiment, monitoring the intelligent puncture needle based on the real-time angle information includes: issuing an alarm message when an angular difference between the real-time angle information and the puncture information exceeds a first set angle value. The alarm message may be one or a combination of a visual alarm and an audible alarm, where the visual alarm includes flashing light, color change of an image, or the like, and the audible alarm includes buzzing, voice prompt, or the like.

In a specific embodiment, a specific form of the alarm message is provided, where the alarm message is a visual alarm. Assuming that the puncture angle is a, the real-time angle information is b, and the first set angle value is c, when b ∈ (a-c, a+c), the alarm message indicates that the puncture needle is displayed in a first set color in the simulation animation; when b∉(a-c, a+c), the puncture needle is displayed in a second set color in the simulation animation. For example, the first set color may be green, and the second set color may be red. When the puncture angle of the puncture needle is within a normal error range, the puncture needle in the virtual animation is green, indicating that the puncture is proceeding normally and can be safely continued. When the puncture angle exceeds the normal error range, the puncture needle in the virtual animation is red, indicating that the puncture angle has deviated and prompting the doctor to correct the deviation.

In this embodiment, a specific structure of the positioning device 3 is provided for achieving its functions. As shown in FIG. 2, the positioning device 3 includes a first rail 31 and a second rail 32. The first rail 31 and the second rail 32 are arranged directly above a scanning table 11 of the medical imaging device 1. The first rail 31 is arranged parallel to a length direction of the scanning table 11. The second rail 32 is perpendicular to the first rail 31 and disposed on the first rail 31, capable of sliding along the first rail 31 when driven by the first rail 31. A laser emitter 33 facing the scanning table 11 is arranged at a lower side of the second rail 32, and the laser emitter 33 is capable of moving along the second rail 32 when driven by the second rail 32. The positioning device 3 further includes a microcontroller and a wireless communication module. The wireless communication module and the laser emitter are electrically connected to the microcontroller, and the microcontroller is configured to control movement of the first rail 31 and the second rail 32.

During installation, the positioning device 3 needs to be arranged facing the scanning table 11 of the medical imaging device 1, as shown in FIG. 2. For example, the positioning device 3 can be fixed on the ceiling directly above the scanning table 11, with the two rails arranged perpendicularly. The first rail 31 is arranged parallel to the length direction of the scanning table 11, and the second rail 32 is disposed on the first rail 31 and capable of sliding along the first rail 31 when driven by the first rail 31. The laser emitter 33 is arranged at the lower side of the second rail 32, and the laser emitter 33 faces the scanning table 11 so that light emitted by the laser emitter 33 can illuminate the patient lying on the scanning table 11. The laser emitter 33 is fixed on the second rail 32. When the second rail 32 moves, the laser emitter 33 can be driven to move along the second rail 32. Furthermore, the positioning device 3 further includes the microcontroller and the wireless communication module. The wireless communication module and the laser emitter 33 are electrically connected to the microcontroller, and the microcontroller is configured to control the movement of the first rail 31 and the second rail 32.

Before use, the positioning device 3 needs to be registered with the medical imaging device 1 to ensure that their coordinates are consistent. During use, the doctor first determines the puncture point position and puncture angle on the host computer 2 based on scanned images. After the puncture point position is determined, spatial coordinates of the puncture point can be determined. At this time, the host computer 2 communicates wirelessly with the microcontroller and transmits the coordinate information to the microcontroller. The microcontroller then controls the movement of the first rail 31 and the second rail 32 according to the coordinate information of the puncture point, adjusting the laser emitter 33 to the coordinates of the puncture point. In this case, the microcontroller controls the laser emitter to emit laser light, and the laser light hits the human body, marking the puncture point position.

The positioning device 3, by providing automated equipment that works in coordination with the medical imaging device 1 and the host computer 2, can automatically mark the puncture point on the human body using the laser emitter 33 according to the spatial coordinate information of the puncture point after the puncture point is determined. It is convenient and accurate to use, avoiding errors caused by manual measurement.

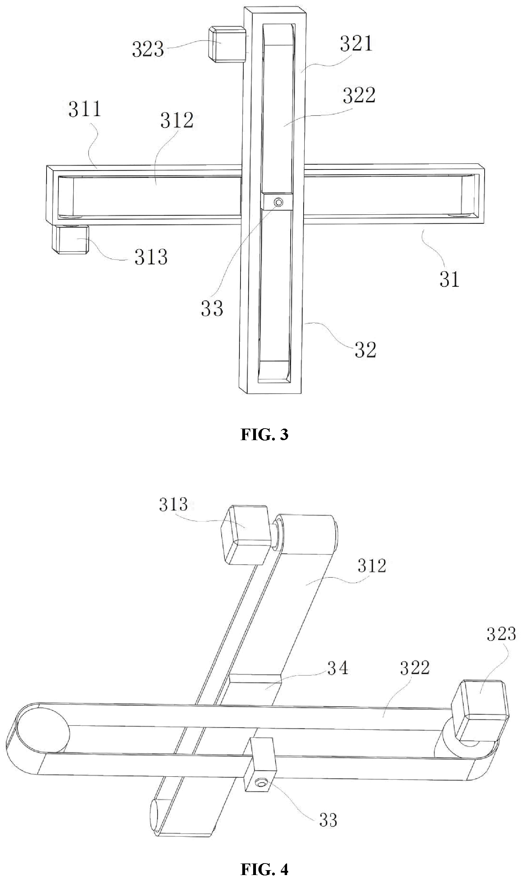

In a specific embodiment, the present disclosure provides a specific structure of the first rail 31. As shown in FIG. 3, the first rail 31 includes a first frame 311 and a first conveyor belt 312. Rotating shafts are provided at two inner ends of the first frame 311, and two ends of the first conveyor belt 312 are fixed to the rotating shafts, respectively. A first motor 313 is provided on the first frame 311 for driving the rotating shafts to rotate. The first motor 313 is electrically connected to the microcontroller. The specific structure of the conveying device enables the microcontroller to control the movement of the first rail 31.

In an embodiment, a plane formed by a centerline of the first frame 311 and a centerline of the scanning table 11 is perpendicular to the scanning table 11. The centerline of the first frame 311 refers to a centerline along its length direction, and the centerline of the scanning table 11 also refers to a centerline along its length direction. By arranging the plane formed by both centerlines to be perpendicular to the scanning table 11, it is ensured that the first frame 311 is positioned directly above the scanning table 11, facilitating the matching of the coordinates of the medical imaging device 1 and the intelligent puncture positioning device 3.

In an embodiment, as shown in FIG. 3, the second rail 32 includes a second frame 321 and a second conveyor belt 322. The second frame 321 is slidably connected to the first frame 311 and is fixedly connected to the first rail 31. Rotating shafts are provided at two inner ends of the second frame 321, and two ends of the second conveyor belt 322 are fixed to the rotating shafts, respectively. A second motor 323 is provided on the second frame 321 for driving the rotating shafts to rotate. The second motor 323 is electrically connected to the microcontroller. Arranging the second frame 321 to be slidably connected to the first frame 311 and fixedly connected to the first rail 31 allows the first frame 311 to bear the weight of the second frame 321 and also allows the first conveyor belt 312 to drive the second frame 321 to move.

This embodiment provides a specific structure for arranging the second frame 321 on the first frame 311. As shown in FIG. 3 and FIG. 4, the second frame 321 is fixed to a connecting plate 34. The connecting plate 34 is slidably connected to the first frame 311 and is fixed on the second conveyor belt 322. The laser emitter 33 is fixed on the second conveyor belt 322.

In an embodiment, the laser emitter 33 is a cross laser emitter, emitting a cross laser spot for easy positioning.

In this embodiment, a specific structure of the intelligent puncture needle 4 is provided for achieving its function. The intelligent puncture needle 4 of this embodiment includes a puncture needle body 41. The puncture needle body 41 is provided with a gyroscope, a Bluetooth module, and a power supply module. The gyroscope and the Bluetooth module are each electrically connected to the power supply module. The gyroscope is configured to detect an angle of the puncture needle body 41 relative to a set reference plane. The Bluetooth module is configured to transmit detected angle information to a mobile phone, the host computer 2, or a tablet computer through wireless communication. The power supply module supplies power to the gyroscope and the Bluetooth module. By integrating a gyroscope, a Bluetooth module, and a power supply module onto an ordinary puncture needle, real-time detection of the puncture angle of the puncture needle can be achieved. Therefore, during puncture, puncture angle deviation can be detected in time, avoiding puncture failure caused by puncture angle deviation, such as puncturing blood vessels, bones or other important parts.

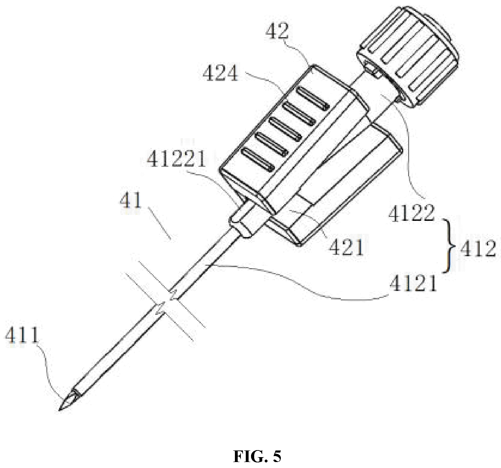

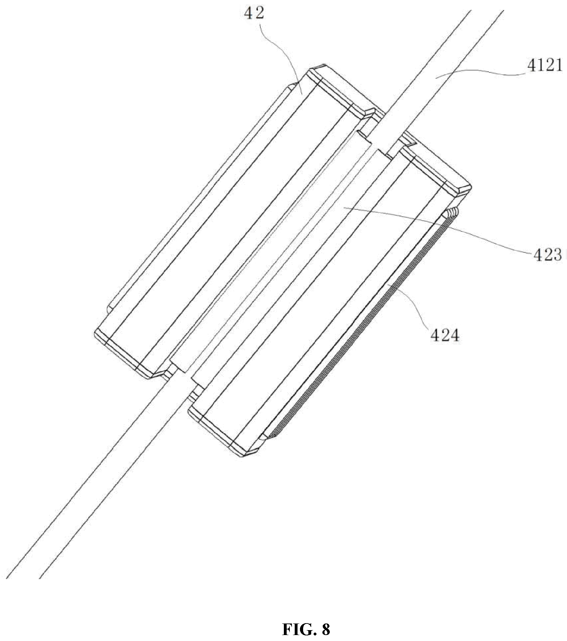

In an embodiment, as shown in FIG. 5 or FIG. 8, the puncture needle body 41 is provided with a housing 42, and the gyroscope, Bluetooth module, and power supply module are all arranged inside the housing 42. Since the agility and lightness of the puncture needle are important during puncture, it needs to be compact and symmetric in weight. To meet this requirement, the cross section of the housing 42 is U-shaped, so that the weight on both sides of the housing can be balanced through reasonable arrangement, and a space for arranging connecting wires is also reserved.

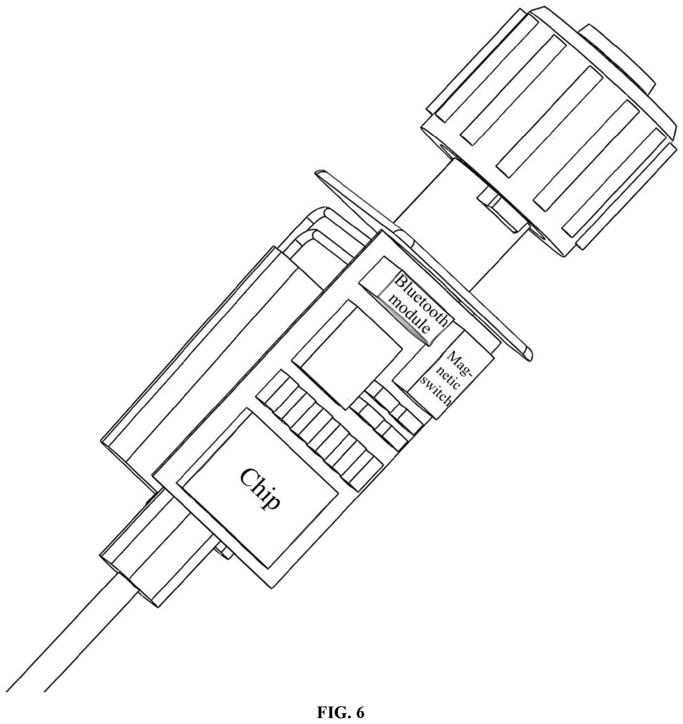

In one embodiment, the gyroscope and the Bluetooth module are integrated on a circuit board and disposed within one side of the housing 42, and the power supply module is disposed within the other side of the housing 42, as shown in FIG. 6. Additionally, a control switch, such as an electromagnetic switch or other chips, may also be integrated on the circuit board. In this way, the electronic components attached to the intelligent puncture needle 4 are divided into two categories: one category includes the gyroscope, Bluetooth module, and the like, which can be integrated on the circuit board and disposed within one side of the housing 42; the relatively heavy power supply module is disposed within the corresponding other side of the housing 42, so as to balance weight and make weights on two sides of the housing 42 substantially balanced. Furthermore, the connecting wires between the power supply module and the circuit board can be arranged within a side wall that connects the two sides of the housing 42.

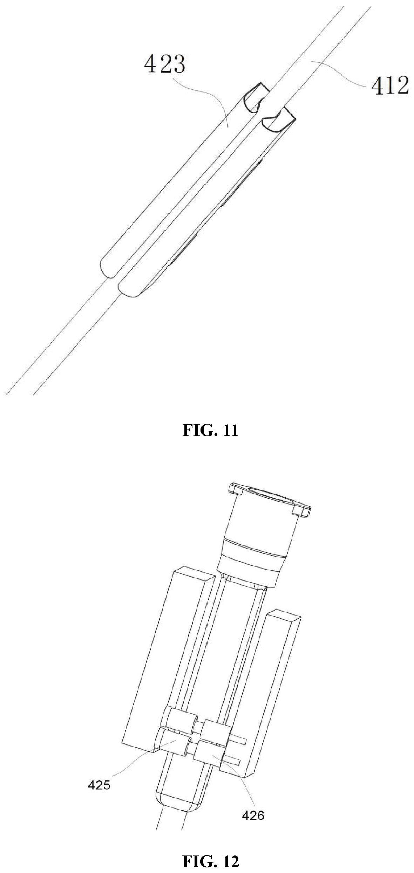

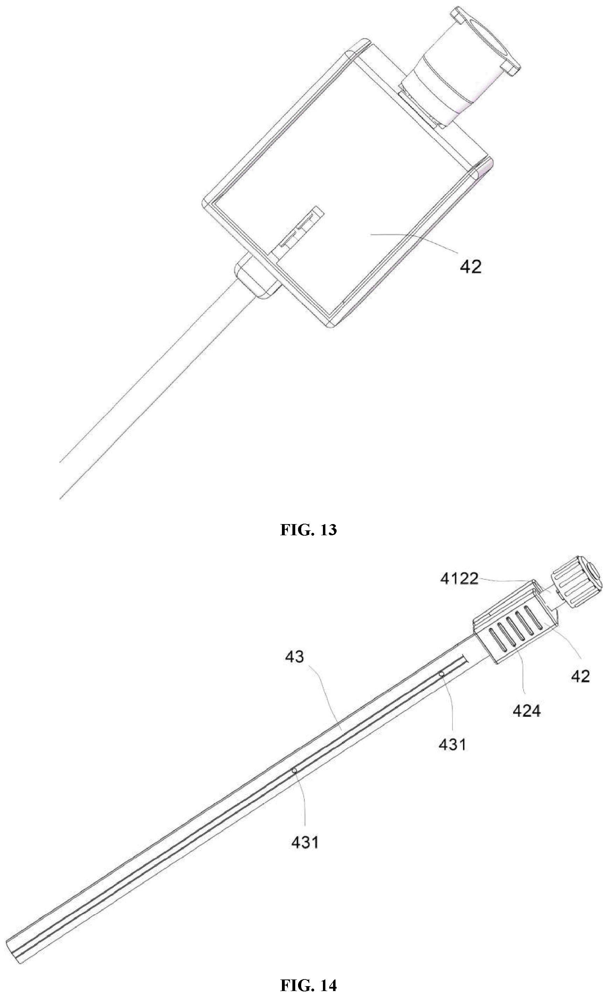

In another embodiment, based on the structure of the housing, another manner of supplying power from a power source to the circuit board is provided. As shown in FIG. 12 and FIG. 13, a first pogo pin plug 425 is electrically connected to the circuit board, and a first pogo pin receptacle 426 is electrically connected to the power source. The first pogo pin plug 425 and the first pogo pin receptacle 426 are arranged opposite each other and come into contact. The housing 42 is provided with a hole at a position corresponding to the contact between the first pogo pin plug 425 and the first pogo pin receptacle 426. A pogo pin connector includes a pogo pin plug and a pogo pin receptacle, is small in size, and saves space, making it suitable for use in the small housing of the present disclosure, as it requires no wiring and conserves interior space. The housing 42 is provided with a hole at a position corresponding to the contact between the first pogo pin plug 425 and the first pogo pin receptacle 426, as shown in FIG. 13. When the intelligent puncture needle is not in use, an insulating paper sheet can be placed between the first pogo pin plug 425 and the first pogo pin receptacle 426 to save power. When the intelligent puncture needle is in use, the insulating paper sheet can be removed to enable power connection. This arrangement allows power on/off without installing a switch chip, saving space inside the housing and being convenient and practical.

In an embodiment, after the circuit board is fixed inside the housing 42, the plane of the circuit board is parallel to the axis of the puncture needle body 41. This design facilitates the gyroscope in detecting the rotation angle of the puncture needle body 41 and simplifies the calculation process. In a specific embodiment, a nine-axis attitude sensor with Bluetooth module may be used to replace the Bluetooth module and the gyroscope. The nine-axis attitude sensor with Bluetooth module is a chip that integrates the functions of a Bluetooth module and a gyroscope. This chip is small in size and powerful, making it suitable for use in the intelligent puncture needle of the present disclosure.

Two specific embodiments are provided below to illustrate the mounting manner and position of the housing 42 on the puncture needle body 41.

As shown in FIG. 5, the puncture needle body 41 generally includes a needle core 411 and a needle hub 412 that are coaxially arranged. Generally, the needle hub 412 includes a metal needle hub rod 4121 and a plastic needle hub handle 4122, which are fixed together by injection molding or adhesive bonding. In a specific embodiment, the housing 42 is fixed to the needle hub handle 4122 of the puncture needle, as shown in FIG. 5.

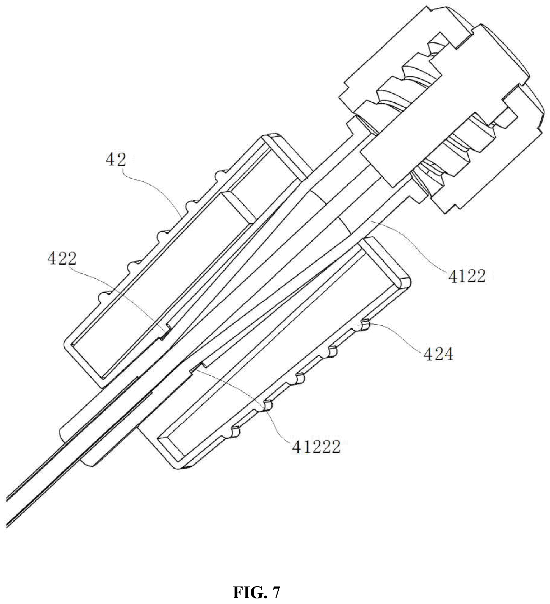

The lower end of the needle hub handle 4122 is relatively thin to facilitate connection with the needle hub rod 4121, while the upper end is relatively thick to facilitate gripping of the puncture needle. The connection between the upper end and the lower end has a gradual transition, becoming thicker from bottom to top. The cross section of the housing 42 is U-shaped. To adapt to the shape of the needle hub handle 4122, the housing 42 is configured to be larger at the top and smaller at the bottom, as shown in FIG. 7.

In an embodiment, as shown in FIG. 5 and FIG. 7, first mounting planes 41221 are symmetrically provided at two lower sides of the needle hub handle 4122, and a clamping groove 41222 is provided at an upper side of each first mounting plane. Second mounting planes 421 matching the first mounting planes 41221 are symmetrically provided at two inner lower sides of the housing 42, and a clamping block 422 matching the clamping groove is provided at an upper side of each second mounting plane 421. By providing the first mounting planes 41221 at the lower end of the needle hub handle and the second mounting planes 421 inside the housing 42, along with the matching clamping grooves 41222 and clamping blocks 422, the housing 42 can be conveniently fixed to the needle hub handle 4122.

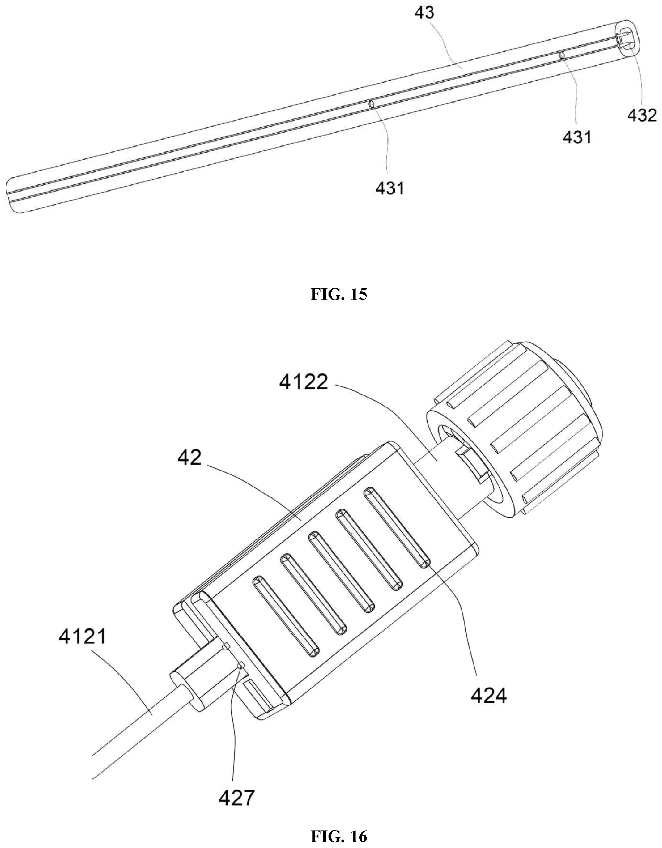

In an embodiment, the intelligent puncture needle further includes a sleeve 43. As shown in FIG. 14, the sleeve 43 is detachably sleeved outside the needle hub 412 of the puncture needle body 41. An FPCB is arranged inside a side wall of the sleeve 43 along a length direction of the sleeve 43. As shown in FIG. 15 and FIG. 16, at least two laser receivers 431 are disposed on the FPCB. Corresponding to the laser receivers 431, the sleeve 43 is provided with holes. A second pogo pin plug 432 is arranged at an end of the sleeve 43, and the second pogo pin plug 432 is electrically connected to the FPCB. Correspondingly, a second pogo pin receptacle 427 is arranged at the lower side of the housing, and the second pogo pin receptacle 427 is electrically connected to the circuit board.

Since the needle hub handle 4122 has a relatively thin lower end and a relatively thick upper end, and the connection between the upper end and the lower end has a gradual transition, the sleeve 43 can be detachably mounted on the needle hub handle 4122. The second pogo pin plug 432 disposed at the end of the sleeve 43 corresponds to the second pogo pin receptacle 427 disposed at the lower side of the housing, such that when the sleeve 43 is fixed on the needle hub handle 4122, the second pogo pin plug 432 is inserted into the second pogo pin receptacle 427, achieving electrical connection. In a specific embodiment, two laser receivers 431 are disposed on the sleeve 43, and the two laser receivers 431 are arranged along a straight line that is parallel to the axis of the puncture needle.

The above arrangement is intended to accomplish registration between the intelligent puncture needle 4 and the medical imaging device 1. Taking a CT device as an example, to register the CT device, a laser beam is emitted from a set zero position. At this time, the doctor holds the intelligent puncture needle so that the laser receivers 431 thereon detect the laser. When the two laser receivers 431 located on the same straight line both detect the laser, a current position of the puncture needle body is set as a zero point of the puncture needle body.

During use, the insulating paper sheet between the first pogo pin plug 425 and the first pogo pin receptacle 426 is removed, and the intelligent puncture needle is powered on. The second pogo pin plug 432 and the second pogo pin receptacle 427 are electrically connected and communicate with each other. When the laser receiver 431 receives a laser signal, it transmits the laser signal to the host computer via the Bluetooth module. When simultaneously receiving the laser signals transmitted by the two laser receivers 431 located on the same straight line, the host computer sets a current position of the puncture needle as a zero point position, achieving automatic registration with the medical imaging device.

In another embodiment, the housing 42 is detachably mounted on the needle hub rod 4121 of the puncture needle. The detachable connection is intended to facilitate the reuse of the intelligent components. The cross section of the housing 42 is U-shaped. To adapt to the shape of the needle hub rod 4121, the housing 42 has a uniform size from top to bottom, as shown in FIG. 8.

As shown in FIG. 8, an elastic clip 423 is provided inside the housing 42 to adapt to puncture needles of different thicknesses. As shown in FIG. 11, the cross section of the elastic clip 423 is U-shaped, formed integrally from a metal sheet. An outer side of the elastic clip 423 is right-angled, and an inner side is arc-shaped, with a connection between the inner and outer sides having an arc-shaped transition. Integral forming from a metal sheet facilitates generating elastic force by mechanical deformation of the metal sheet, thereby securing the puncture needle. To adapt to the shape of the housing 42, the cross section of the elastic clip 423 is also a U-shaped, with the outer side of the elastic clip 423 being right-angled to conform to the shape of the inner side of the housing, while the inner side of the elastic clip 423 is arc-shaped to conform to the shape of the puncture needle.

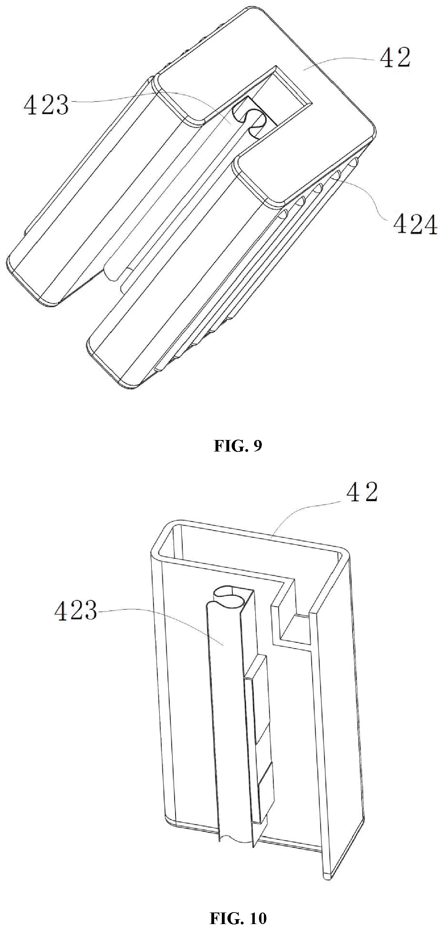

As shown in FIG. 9 and FIG. 10, a groove is provided on a rear side of the elastic clip 423, and correspondingly, a protrusion is provided on the inner side of the housing 42. The elastic clip 423 is fixed by cooperation between the protrusion and the groove. To prevent slipping during gripping, anti-slip protrusions 424 are provided on the outer side of the housing 42.

In the prior art, the step-by-step needle insertion method involves scanning while puncturing to determine whether the puncture path is correct and to avoid mispuncture. When scanning is performed during puncture, to avoid radiation, the patient is scanned alone in the scanning room. At this time, the puncture needle is difficult to secure since it has been partially inserted into the human body, and it cannot be determined whether the puncture direction deviates due to body movement or other reasons during scanning.

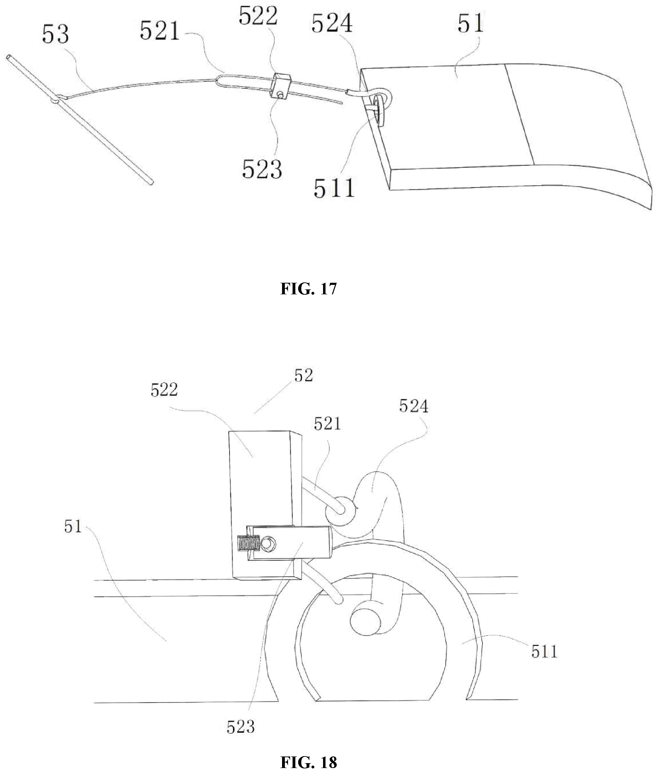

This embodiment provides a puncture needle fixation device 5 for use when the puncture needle has been partially inserted into the body during the puncture process. As shown in FIG. 17, the puncture needle fixation device 5 includes a silicone fixation plate 51, an adjustment device 52, and a hook 53. The adjustment device 52 includes an adjustment cord 521 and an adjustment buckle. The adjustment buckle includes a lock body 522 and the adjustment cord 521. One end of the adjustment cord 521 is fixedly connected to the lock body 522 and passes through the lock body 522 to connect to the silicone fixation plate 51. Another end of the adjustment cord 521 passes through the lock body 522 and is adjustably connected to the lock body 522. One end of the hook 53 is slidably connected to a bending portion of the adjustment cord 521.

The puncture needle fixation device includes the silicone fixation plate 51. The silicone fixation plate 51 is made of medical-grade silicone material, which is skin-friendly, soft, and capable of conforming to human skin without being affected by skin shape. By selecting an appropriate thickness and size, the silicone fixation plate 51 can support the puncture needle through its own weight and friction with the skin. The hook 53 is configured to hook the puncture needle. The hook 53 and the silicone fixation plate 51 are connected via the adjustment device 52. The adjustment device 52 is configured to adjust a distance between the hook 53 and the silicone fixation plate 51 and support the hook 53, thereby adjusting a fixation angle of the puncture needle body 41.

The adjustment device 52 includes the adjustment cord 521 and the lock body 522. Both ends of the adjustment cord 521 pass through the lock body 522, where one end is fixed to the lock body 522 and connected to the silicone fixation plate 51, while the other end is in an adjustable state, that is, a length of a free end outside the lock body 522 can be adjusted, thereby adjusting the distance between the hook 53 and the silicone fixation plate 51. One end of the hook 53 is slidably connected to the bending portion of the adjustment cord 521, ensuring that the one end of the hook 53 is always located near the midpoint of the bending portion.

During use, the hook 53 is first hooked onto the puncture needle to be fixed. Then the hook 53 and the adjustment device 52 are stretched, the silicone fixation plate 51 is adhered to an appropriate position on the skin of the patient, and the length of the free end of the adjustment cord 521 is finely adjusted to a suitable position to secure the puncture needle. Additionally, the position where the hook (53) hooks onto the puncture needle can be adjusted, thereby adjusting a fixation direction of the puncture needle. The puncture needle fixation device can secure the puncture needle during puncture scanning without affecting the scanning process, and is convenient and quick to use.

A specific embodiment provides a specific structure of the adjustment device. As shown in FIG. 18, the lock body 522 is correspondingly provided with a fixing hole and an adjustment hole. One end of the adjustment cord 521 passes through the fixing hole to connect to the silicone fixation plate 51, and the adjustment cord 521 is fixedly connected to the fixing hole. The other end of the adjustment cord 521 passes through the adjustment hole. The adjustment hole is provided with an adjustment button 523. When the adjustment button 523 is pressed, the adjustment cord 521 can move back and forth along the adjustment hole; when the adjustment button 523 is released, the adjustment cord 521 is fixed within the adjustment hole.

In one embodiment, as shown in FIG. 17, a hanging ring 511 is provided at one side of the silicone fixation plate 51, and a fixing hook 524 is provided at the fixed end of the adjustment cord 521. The fixing hook 524 hooks into the hanging ring 511. As shown in FIG. 18, a through hole is provided at a lower end of the adjustment button 523. After the adjustment button 523 is mounted on the lock body, the through hole corresponds to the adjustment hole. The adjustment button 523 is fixed within the lock body 522 by a spring. This structure is simple and easy to operate.

In the present disclosure, the intelligent puncture needle 4 is used. Since the intelligent puncture needle integrates an intelligent chip, the weight of the needle hub handle 4122 is increased. During step-by-step needle insertion, if the puncture needle is not fixed, gravity can significantly affect the puncture direction. Therefore, the puncture needle fixation device 5 is provided for fixing the intelligent puncture needle 4. Moreover, because the real-time angle information of the puncture needle can be obtained, it can be detected whether the puncture angle has deviated during the step-by-step needle insertion scanning, ensuring the accuracy of subsequent puncture.

Additionally, a dedicated mechanical fixture, such as a six-axis mechanical fixture, can be used to fix the intelligent puncture needle 4, making adjustments flexible and convenient. The mechanical fixture can be made of carbon steel to avoid interfering with the normal operation of the medical imaging device. Unlike robotic arms used in optical navigation and magnetic navigation systems, the mechanical fixture used in the intelligent puncture navigation system does not need to display angles nor have a guiding puncture function, but only needs to serve a supporting and fixing function.

To simplify calculation and improve coordination among components, the present disclosure adopts a three-in-one coordinate system. Specifically, the positioning device 3, the intelligent puncture needle 4, and the medical imaging device 1 share a common coordinate system, all using the coordinate system of the medical imaging device 1. When a medical imaging device is installed, an installation environment, such as a horizontal plane (floor) and installation orientation, is adjusted. Therefore, it is most reasonable to match the coordinate systems of the positioning device 3 and the intelligent puncture needle 4 with the coordinate system of the medical imaging device 1. After registration, accurate mapping from physical entities to virtual entities is achieved, and then the free observation characteristics of the virtual space are used to guide the physical puncture service.

Based on this, the present disclosure also provides another application example, providing an intelligent puncture navigation method applied to the intelligent puncture navigation system described above. The method includes:
1). Establish an OC coordinate system based on a medical imaging device, and establish an OL coordinate system based on a positioning device, where an x-axis direction and a z-axis direction of the OC coordinate system are the same as an x-axis direction and a z-axis direction of the OL coordinate system.
2). Determine a mapping relationship from the OC coordinate system to the OL coordinate system.
3). Acquire a three-dimensional scanned image of a target puncture tissue using the medical imaging device.
4). Determine puncture information by using a host computer based on the three-dimensional scanned image, where the puncture information includes a puncture point position, a puncture angle, and a needle insertion depth.
5). Establish an OP coordinate system based on a position of the target puncture tissue, and determine a mapping relationship from the OP coordinate system to the OC coordinate system, where an origin of the OP coordinate system is located at a needle insertion point position, and the needle insertion point position is a position where the positioning device forms a mark on a skin surface of the target puncture tissue according to the puncture information.
6). Determine a mapping relationship from the OP coordinate system to the OL coordinate system based on a mapping relationship from the OL coordinate system to the OC coordinate system and the mapping relationship from the OP coordinate system to the OC coordinate system.
7). Establish an OU coordinate system based on a position of the target puncture tissue in a virtual space, and determine a mapping relationship from the OC coordinate system to the OU coordinate system, where an origin of the OU coordinate system is a needle insertion point position on the target puncture tissue in the virtual space.
8). Establish a one-dimensional ON coordinate system based on an intelligent puncture needle, where an origin of the one-dimensional ON coordinate system is a needle tip position of the puncture needle.
9). Perform a puncture process simulation in the virtual space based on the puncture information to determine a virtual puncture needle tip position of the puncture needle.
10). Determine real-time angle information based on the virtual puncture needle tip position of the puncture needle.
11). Guide puncture of the intelligent puncture needle based on the real-time angle information.

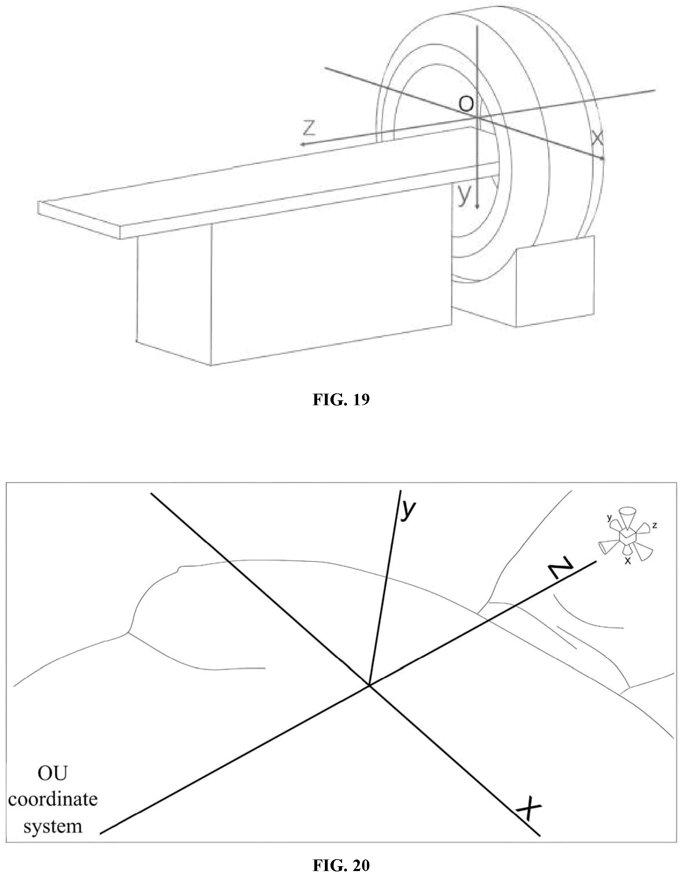

The specific implementation principle is described below using a CT scanner as an example, involving the relationships and mutual conversions among a plurality of coordinate systems, where the OC coordinate system is shown in FIG. 19, and the OU coordinate system is shown in FIG. 20. Specific steps are as follows:
(1) Cartesian coordinate system of the CT scanner: C(x, y, z), OC(xC, yC, zC), with the origin located at a point in space where the CT cross laser directly hits when the CT table is reset.
(2) Cartesian coordinate system of the positioning device 3: L(x, z), OL(xL, zL), with the origin located at the center of the positioning device 3.
(3) Left-handed three-dimensional Cartesian coordinate system of the virtual space: U(x, y, z), OU(xU, yU, zU), with the origin located at the spatial position of the needle tip in the virtual space.
(4) Right-handed three-dimensional Cartesian coordinate system of the patient: P(x, y, z), OP(xP, yP, zP), with the origin located at the spatial position of the skin needle insertion point.
(5) One-dimensional coordinate axis of the intelligent puncture needle 4: N(s), ON(sN), with the origin located at the needle tip.

Steps for conversion between coordinate systems are as follows:
Step 1: Establish the OC coordinate system based on the CT scanner, as shown in FIG. 19.
Step 2: Establish the OL coordinate system, where the xL axis is parallel to and has the same direction as the xC axis, and the zL axis is parallel to and has the same direction as the zC axis.
Step 3: Establish the mapping from the OL coordinate system to the OC coordinate system. After the positioning device 3 is installed, the host computer 2 controls the positioning device 3 to return to the origin of the coordinate system, the laser emitter 33 is turned on, and a debug board is placed on the CT table so that the laser coincides with the metal cross. Then, with the position of the debug board kept unchanged, a CT scan is performed on the debug board, and the coordinates OC (x0, y0, z0) of the center point of the metal cross on the debug board are read in the CT software, thereby establishing the mapping relationship from the OC coordinate system to the OL coordinate system, where the coordinates of OL(0,0,0) in the CT coordinate system are OC(x0, y0+h, z0), and h is a constant and may not be measured. After the CT table moves a distance s1 in the negative direction of the z-axis, the coordinates of OL(0,0,0) in the OC coordinate system remain unchanged, still being OC(x0, y0+h, z0).
Step 4: Establish the OP coordinate system, and establish the mapping from the OP coordinate system to the OC coordinate system. After the CT scan is completed, the CT table returns to its initial position. At this time, the OP coordinate system completely coincides with the OC coordinate system, and the coordinates of OP(xP, yP, zP) in the CT coordinate system are OC(xC, yC, zC). After the CT table moves a distance s1 in the negative direction of the z-axis, the coordinates of OP(xP, yP, zP) in the CT coordinate system are OC(xC, yC, zC-s1).

After the CT scan is completed, an optimal puncture path is planned according to the lesion position while avoiding important tissues and organs around the lesion; a skin puncture point and a puncture angle are determined. The coordinates (xC1, yC1, zC1) of a target puncture point P1 as well as the coordinates (xC2, yC2, zC2) of the corresponding skin needle insertion point P2 are found on CT images, and a roll angle θ and a pitch angle α during puncture from P2 to P1 are measured, both defined as angles between the central axis of the needle body and the negative direction of the yC axis, with values always being non-negative and ranging from [0, 180] degrees. θ is measured in a CT cross-sectional image, and α is measured in a CT sagittal image.

After the CT table moves a distance s1 in the negative direction of the z-axis, the coordinates of P1 in the OC coordinate system are (xC1, yC1, zC1-s1), the coordinates of P2 in the OC coordinate system are (xC2, yC2, zC2-s1), and the roll angle θ and pitch angle α remain unchanged.

Step 5: Establish the mapping from the OP coordinate system to the OL coordinate system. After the CT table moves a distance s1 in the negative direction of the z-axis, the coordinates of the skin needle insertion point P2 in the OC coordinate system are (xC2, yC2, zC2-s1), and its corresponding coordinates in the OL coordinate system are OL(xC2-x0, zC2-s1-z0). The coordinates OL(xC2-x0, zC2-s1-z0) are used to operate the positioning device 3 to move the center of the laser emitter 33 to the planned skin needle insertion point.

Step 6: Establish the OU coordinate system, and establish the mapping from the OC coordinate system to the OU coordinate system. The OU coordinate system is established when Unity3D is started. The origin of the OU coordinate system is at the skin needle insertion point. The xU axis is parallel to and in the same direction as the xC axis. The yU axis is parallel to the yC axis but oriented in the opposite direction. The zU axis is parallel to the zC axis but oriented in the opposite direction. During calculation of the roll angle θ and the pitch angle α in the OU coordinate system, the angles between the central axis of the needle body and the positive direction of the yU axis are used, with values always being non-negative and ranging from [0, 180] degrees. In the OU coordinate system, the origin of the OU coordinate system always coincides with the skin needle insertion point. Since the OU coordinate system has the same x-axis direction as the OC coordinate system, but opposite y-axis and z-axis directions, the positional deviation of the target puncture point P1 from the skin needle insertion point P2 in the OU coordinate system is Δx, -Δy, -Δz, and thus the coordinates of P1 in the OU coordinate system are OU(xC1-xC2, yC2-yC1, zC2-zC1).

Step 7: Establish the ON coordinate axis, and establish the mapping from the ON coordinate axis to the OU coordinate system. The ON coordinate axis is directly photolithographically etched on the needle body, with the needle tip as the coordinate origin, the central axis of the needle body as the s-axis, and the direction toward the needle tail as positive, such that any given point on the needle body is represented by the coordinate ON(sN). The function of the ON coordinate axis is to guide the needle insertion depth, and the depth value is directly measured from the CT image. During the puncture process, there are two CT scans to verify the direction. Before scanning, the scale value sN on the needle body can be read and input into the host computer software to obtain current relative positions of the puncture needle and the target puncture point. However, since the puncture needle may tilt, the spatial position of the needle insertion point may change. Therefore, the spatial position of the puncture needle displayed in the host computer software may have a certain deviation and is for reference only. The specific position is subject to the CT scan image (this error does not affect the accuracy of the final puncture). Under the ideal condition that the position of the skin needle insertion point P2 remains unchanged, the coordinates of the puncture needle tip in the OU coordinate system are OU(±tanθ*sN/sqrt(1+tan2α+tan2θ), -sN/sqrt(1+tan2α+tan2θ), ±tanα*sN/sqrt(1+tan2α+tan2θ)), and the central axis of the needle body passes through OU(0,0,0).

The values that need to be entered into the host computer software are as follows: In the system debugging phase, x0, y0, and z0 are entered (recording the relative positional relationship between the CT scanner and the positioning device). In the use phase, z1, xC1, yC1, zC1, xC2, yC2, zC2, and the needle insertion depth sN are entered.

The spatial posture of the needle body is calculated based on quaternion data generated by a six-axis attitude sensor, and the quaternion data is transmitted to the host computer via Bluetooth. The host computer software calculates and displays the spatial posture of the needle body based on the Unity3D engine. When the Unity3D engine is initialized, the central axis of the needle body is parallel to the z-axis, and the needle tip points toward the negative direction of the z-axis. Since the six-axis attitude sensor chip has its negative y-axis direction pointing toward the needle tip, its negative z-axis direction pointing toward the center of the earth, and employs a right-handed coordinate system, the quaternion output by this chip, when input into the Unity3D left-handed coordinate system, needs to be converted from (w, x, y, z) to (x, z, y, -w).

The display of the needle body posture is based on the Quaternion.RotateTowards function of Unity3D, which can achieve the rotation of an object from an initial position to a given quaternion position. In this software, the rotation from the quaternion (0, 0, 0, 0) to (x, z, y, -w) is achieved.

The calculation of the roll angle θ and pitch angle α of the needle body is also based on quaternions. The specific method is to left-multiply an initial unit vector v1 (0, 0, 1) of the central axis of the needle body in the OU coordinate system by the rotation from (0,0,0,0) to (x, z, y, -w), to obtain a rotated vector v2 (x2, y2, z2) of the central axis of the needle body. Then, the roll angle θ = 90 - arcsin(y2/sqrt(y2*y2+x2*x2))/π*180, and the pitch angle α = 90 - arcsin(y2/sqrt(y2*y2+z2*z2))/π* 180.

According to the above description, the position of the target puncture point P1 in the OU coordinate system is OU(xC1-xC2, yC2-yC1, zC2-zC1). The position of the needle tip in the OU coordinate system is OU(±tanθ*sN/sqrt(1+tan2α+tan2θ), -sN/sqrt(1+tan2α+tan2θ), ±tanα*sN/sqrt(1+tan2α+tan2θ)). The rotation of the needle body always occurs around the skin needle insertion point P2, which has the coordinates OU(0, 0, 0) in the OU coordinate system.

The technical characteristics of the above embodiments can be employed in arbitrary combinations. To provide a concise description of these embodiments, all possible combinations of all the technical characteristics of the above embodiments may not be described; however, these combinations of the technical characteristics should be construed as falling within the scope defined by the specification as long as no contradiction occurs.

Several examples are used herein for illustration of the principles and implementations of the present disclosure. The description of the above embodiments is only used to help understand the method of the present disclosure and the core principles thereof. In addition, those of ordinary skill in the art can make various modifications in terms of the specific implementations and scope of application in accordance with the teachings of the present disclosure. In conclusion, the content of the specification shall not be construed as a limitation to the present disclosure.

## Claims

1. An intelligent puncture navigation system, comprising: a medical imaging device (1), a host computer (2), a positioning device (3), and an intelligent puncture needle (4);
wherein the medical imaging device (1) is configured to acquire a three-dimensional scanned image of a target puncture tissue; and the medical imaging device (1) is a computed tomography (CT) or magnetic resonance imaging (MRI) device;
the host computer (2) is configured to determine puncture information according to the three-dimensional scanned image, wherein the puncture information comprises a puncture point position, a puncture angle, and a needle insertion depth;
the positioning device (3) is configured to form a mark on a skin surface of the target puncture tissue according to the puncture information, wherein the mark is a needle insertion point position;
the intelligent puncture needle (4) is configured to perform puncture according to the mark and the puncture information, and acquire real-time angle information of the intelligent puncture needle (4);
the host computer (2) is further configured to monitor the intelligent puncture needle (4) according to the real-time angle information;
the positioning device (3), the intelligent puncture needle (4), and the medical imaging device (1) share a coordinate system;
the positioning device (3) comprises a first rail (31) and a second rail (32); the first rail (31) and the second rail (32) are arranged directly above a scanning table (11) of the medical imaging device (1), wherein the first rail (31) is arranged parallel to a length direction of the scanning table (11); the second rail (32) is perpendicular to the first rail (31) and disposed on the first rail (31), capable of sliding along the first rail (31) when driven by the first rail (31); a laser emitter (33) facing the scanning table (11) is arranged at a lower side of the second rail (32), and the laser emitter (33) is capable of moving along the second rail (32) when driven by the second rail (32);
the positioning device (3) further comprises a microcontroller and a wireless communication module; the wireless communication module and the laser emitter (33) are electrically connected to the microcontroller, and the microcontroller is configured to control movement of the first rail (31) and the second rail (32);
the host computer (2) communicates wirelessly with the microcontroller and transmits coordinate information of a puncture point to the microcontroller; the microcontroller then controls the movement of the first rail (31) and the second rail (32) according to the coordinate information of the puncture point, adjusting the laser emitter (33) to a position corresponding to the coordinate information; the microcontroller controls the laser emitter (33) to emit laser light, and the laser light hits a human body, marking the puncture point position;
the intelligent puncture needle (4) comprises a sleeve (43) detachably sleeved outside a needle hub (412) of a puncture needle body (41); a flexible printed circuit board (FPCB) is arranged inside a side wall of the sleeve (43) along a length direction of the sleeve (43); at least two laser receivers (431) are disposed on the FPCB; corresponding to the laser receivers (431), the sleeve (43) is provided with holes; a second pogo pin plug (432) is arranged at an end of the sleeve (43); the second pogo pin plug (432) is electrically connected to the FPCB; correspondingly, a second pogo pin receptacle (427) is arranged at a lower side of a housing (42), and is electrically connected to a circuit board;
the medical imaging device (1) emits a laser beam from a set zero position; the laser receivers (431) on the intelligent puncture needle (4) detect the laser beam; when the two laser receivers (431) located on a same straight line both detect the laser beam, a current position of the puncture needle body is set as a zero point of the puncture needle body; and
when the intelligent puncture needle (4) is powered on, the second pogo pin plug (432) and the second pogo pin receptacle (427) are electrically connected and communicate with each other; when receiving laser signals, the laser receivers (431) transmit the laser signals to the host computer (2) via a Bluetooth module; when simultaneously receiving the laser signals from the two laser receivers (431) on the same straight line, the host computer (2) sets a current position of the puncture needle body (41) as a zero point position, achieving automatic registration with the medical imaging device (1);
the intelligent puncture navigation system further comprises a puncture needle fixation device (5); the puncture needle fixation device (5) comprises a silicone fixation plate (51), an adjustment device (52), and a hook (53); the adjustment device (52) comprises an adjustment cord (521) and an adjustment buckle; the adjustment buckle comprises a lock body (522); one end of the adjustment cord (521) is fixedly connected to the lock body (522) and passes through the lock body (522) to connect to the silicone fixation plate (51); another end of the adjustment cord (521) passes through the lock body (522) and is adjustably connected to the lock body (522); and one end of the hook (53) is slidably connected to a bending portion of the adjustment cord (521);
the silicone fixation plate (51) supports the puncture needle body (41) through gravity and friction with skin; and
the hook (53) is configured to hook the puncture needle body (41); the hook (53) and the silicone fixation plate (51) are connected via the adjustment device (52), wherein the adjustment device (52) is configured to adjust a distance between the hook (53) and the silicone fixation plate (51) and support the hook (53), thereby adjusting a fixation angle of the puncture needle body (41).

2. The intelligent puncture navigation system according to claim 1, wherein the host computer (2) is further configured to form a simulation animation on the three-dimensional scanned image based on the real-time angle information.

3. The intelligent puncture navigation system according to claim 1, wherein the monitoring the intelligent puncture needle (4) based on the real-time angle information comprises: issuing an alarm message when an angular difference between the real-time angle information and the puncture information exceeds a first set angle value.

4. The intelligent puncture navigation system according to claim 3, wherein assuming that the puncture angle is a, the real-time angle information is b, and the first set angle value is c, when b ∈ (a-c, a+c), the alarm message indicates that the puncture needle body is displayed in a first set color in the simulation animation; and when b∉(a-c, a+c), the puncture needle body is displayed in a second set color in the simulation animation.

5. The intelligent puncture navigation system according to claim 1, wherein the puncture needle body (41) is provided with a gyroscope, a Bluetooth module, and a power supply module, and the gyroscope and the Bluetooth module are each electrically connected to the power supply module.

6. The intelligent puncture navigation system according to claim 3, further comprising a display device configured to display the real-time angle information in real time.

7. The intelligent puncture navigation system according to claim 1, wherein the first rail (31) comprises a first frame (311) and a first conveyor belt (312); rotating shafts are provided at two inner ends of the first frame (311), and two ends of the first conveyor belt (312) are fixed to the rotating shafts, respectively; a first motor (313) is provided on the first frame (311) for driving the rotating shafts to rotate; the first motor (313) is electrically connected to the microcontroller; and the microcontroller controls the movement of the first rail (31);
the second rail (32) comprises a second frame (321) and a second conveyor belt (322); the second frame (321) is slidably connected to the first frame (311) and is fixedly connected to the first rail (31); rotating shafts are provided at two inner ends of the second frame (321), and two ends of the second conveyor belt (322) are fixed to the rotating shafts, respectively; a second motor (323) is provided on the second frame (321) for driving the rotating shafts to rotate; the second motor (323) is electrically connected to the microcontroller; and
the laser emitter (33) is fixed to the second conveyor belt (322).

8. The intelligent puncture navigation system according to claim 1, wherein the puncture needle body (41) comprises a needle core (411) and the needle hub (412) that are coaxially arranged; the needle hub (412) comprises a needle hub rod (4121) and a needle hub handle (4122) fixed together; and the housing (42) is fixed to the needle hub handle (4122) of the puncture needle body (41).

9. The intelligent puncture navigation system according to claim 8, wherein first mounting planes (41221) are symmetrically provided at two lower sides of the needle hub handle (4122), and a clamping groove (41222) is provided at an upper side of each of the first mounting planes (41221); second mounting planes (421) matching the first mounting planes (41221) are symmetrically provided at two inner lower sides of the housing (42), and a clamping block (422) matching the clamping groove (41222) is provided at an upper side of each of the second mounting planes (421).

10. An intelligent puncture navigation method, applied to the intelligent puncture navigation system according to any one of claims 1 to 9, wherein the intelligent puncture navigation method comprises:
establishing an OC coordinate system based on a medical imaging device, and establishing an OL coordinate system based on a positioning device, wherein an x-axis direction and a z-axis direction of the OC coordinate system are the same as an x-axis direction and a z-axis direction of the OL coordinate system;
determining a mapping relationship from the OC coordinate system to the OL coordinate system;
acquiring a three-dimensional scanned image of a target puncture tissue using the medical imaging device;
determining puncture information by using a host computer based on the three-dimensional scanned image, wherein the puncture information comprises a puncture point position, a puncture angle, and a needle insertion depth;
establishing an OP coordinate system based on a position of the target puncture tissue, and determining a mapping relationship from the OP coordinate system to the OC coordinate system, wherein an origin of the OP coordinate system is located at a needle insertion point position, and the needle insertion point position is a position where the positioning device forms a mark on a skin surface of the target puncture tissue according to the puncture information;
determining a mapping relationship from the OP coordinate system to the OL coordinate system based on a mapping relationship from the OL coordinate system to the OC coordinate system and the mapping relationship from the OP coordinate system to the OC coordinate system;
establishing an OU coordinate system based on a position of the target puncture tissue in a virtual space, and determining a mapping relationship from the OC coordinate system to the OU coordinate system, wherein an origin of the OU coordinate system is a needle insertion point position on the target puncture tissue in the virtual space;
establishing a one-dimensional ON coordinate system based on an intelligent puncture needle, wherein an origin of the one-dimensional ON coordinate system is a needle tip position of the puncture needle;
performing a puncture process simulation in the virtual space based on the puncture information to determine a virtual puncture needle tip position of the puncture needle;
determining real-time angle information based on the virtual puncture needle tip position of the puncture needle; and
guiding puncture of the intelligent puncture needle based on the real-time angle information.

11. The intelligent puncture navigation method according to claim 10, wherein the virtual puncture needle tip position of the puncture needle is OU(±tanθ*sN/sqrt(1+tan2α+tan2θ), -sN/sqrt(1+tan2α+tan2θ), ±tanα*sN/sqrt(1+tan2α+tan2θ)), and a central axis of a needle shaft of the puncture needle passes through the origin of the OU coordinate system,
wherein θ and α represent a roll angle and a pitch angle from the needle insertion point position on the target puncture tissue to the puncture point position, respectively, and sN is the needle insertion depth.
